# FASCICULE DE BREVET EUROPEEN

(11) **EP 1 155 688 B1**
(45) Date de publication et mention de la délivrance du brevet: **23.07.2003**
(21) Numéro de dépôt: 01401162.1
(22) Date de dépôt: 04.05.2001
(51) Int. Cl.: A61K 7/48, A61K 7/025

(54) **Utilisation en cosmétique d'au moins un organopolysiloxane en tant que gélifiant et composition cosmétique le contenant**
Verwendung in der Kosmetik von einem Organopolysiloxan als Geliermittel sowie eine kosmetischen Zusammensetzung dieses enthaltend
Use in cosmetics of at least a organopolysiloxane as a gelling agent and a cosmetic composition containing said product

(30) Priorité: 09.05.2000 FR 0005876
(43) Date de publication de la demande: 21.11.2001
(73) Titulaire: L'OREAL, 75008 Paris (FR)
(72) Inventeur: Mondet, Jean, 93600 Aulnay-sous-Bois (FR)
(74) Mandataire: Dossmann, Gérard

(56) Documents cités:
- EP-A- 0 445 700
- EP-A- 0 706 790
- EP-A- 0 751 170
- EP-A- 0 850 644
- EP-A- 0 979 643
- DE-A- 19 707 970
- FR-A- 2 765 800
- FR-A- 2 773 485
- US-A- 4 744 978
- US-A- 5 160 732
- US-A- 5 280 019
- ABED S ET AL: "SUPRAMOLECULAR ASSOCIATION OF ACID TERMINATED POLYDIMETHYLSIOXANES.1. SYNTHESIS AND CHARACTERIZATION" POLYMER BULLETIN,DE,SPRINGER VERLAG. HEIDELBERG, vol. 39, no. 3, 1 septembre 1997 (1997-09-01), pages 317-324, XP000721177 ISSN: 0170-0839

## Description

La présente invention concerne l'utilisation en cosmétique, en tant que gélifiant, d'au moins un organopolysiloxane, linéaire ou cyclique, qui comporte au moins deux motifs organosiloxy et au moins deux groupes latéraux et/ou terminaux capables d'établir chacun au moins une liaison hydrogène avec un ou des groupes partenaires. Elle concerne aussi une composition cosmétique comprenant, dans un milieu cosmétiquement acceptable, au moins un tel organopolysiloxane.

En cosmétique, on cherche à obtenir des compositions qui, appliquées sur la peau, y compris celle des paupières, ainsi que sur les lèvres et les phanères, forment des dépôts, par exemple, filmogènes, ayant un ou plusieurs effets cosmétiques et/ou de soin particuliers souhaités.

Ainsi, pour les compositions de maquillage telles que des rouges à lèvres, des mascaras et des "eye-liners", on cherche à obtenir des compositions qui forment des dépôts ayant des effets de coloration et/ou de brillance appropriés. Pour les compositions incluant un actif de soin telles que les produits hydratants, les déodorants et les anti-transpirants, on cherche en particulier à obtenir l'effet optimal de l'actif de soin présent dans la composition.

Dans tous les cas, on cherche également à obtenir la durée la plus longue du ou des effets cosmétiques et/ou de soin. Par exemple, pour les rouges à lèvres, les mascaras et les eye-liners, il est important d'obtenir une tenue prolongée de la coloration et/ou de la brillance ; pour les fonds de teint, les poudres, les fards à joues, les ombres à paupières et les maquillages de corps, il est important d'obtenir un effet mat persistant et durable malgré les frottements ou la sécrétion de sébum ou de sueur ; et pour les compositions incluant un actif de soin, une activité la plus longue possible de l'actif.

On a proposé d'incorporer des huiles de silicone dans des compositions cosmétiques. L'incorporation de ces huiles de silicone dans les compositions apporte aux dépôts des propriétés d'hydrophobicité, de brillance et de toucher non gras, mais les dépôts obtenus résistent mal aux agents externes comme la sueur ou le sébum, et en particulier aux agressions mécaniques comme le frottement.

Des polysiloxanes comportant des motifs amides et pouvant comporter des groupes capables d'établir des liaisons hydrogène, utilisés comme gélifiants des huiles de silicone de compositions cosmétiques, sont décrits dans le brevet WO 99/06473 de Colgate-Palmolive. On obtient ainsi des compositions généralement solides, transparentes ou translucides.

Le brevet US N° 5 919 441 de Colgate-Palmolive décrit une composition cosmétique à base d'un composant fluide comprenant au moins une silicone volatile ou non, et d'au moins un gélifiant. Ce gélifiant est un polymère contenant à la fois des motifs organosiloxy et des groupes formant des liaisons hydrogène qui sont choisis parmi les groupes ester, uréthanne, urée, thiourée, amide et leurs combinaisons. L'emploi d'un tel gélifiant conduit, en particulier, à des compositions solides, de préférence transparentes ou translucides.

Selon l'invention, on a trouvé que l'utilisation d'au moins un organopolysiloxane spécifique, différent de ceux décrits dans les brevets cités ci-dessus, permet d'obtenir la gélification d'un milieu cosmétiquement acceptable et entraîne une élimination possible des agents de gélification habituels.

Ces organopolysiloxanes spécifiques, linéaires ou cycliques, comportent au moins deux motifs organosiloxy et au moins deux groupes latéraux et/ou terminaux capables d'établir chacun au moins une liaison hydrogène avec un ou des groupes partenaires. Ils ont déjà été décrits dans le brevet FR 2 708 272 de Rhône-Poulenc comme adhésifs. Cependant, ils n'ont jamais été utilisés en cosmétique.

Par groupe partenaire, on entend tout groupe latéral et/ou terminal porté par une autre molécule dudit organopolysiloxane, capable de former au moins une liaison hydrogène avec le groupe latéral et/ou terminal dudit organopolysiloxane. Ce groupe partenaire est identique ou non au groupe latéral et/ou terminal avec lequel il forme au moins une liaison hydrogène.

Par milieu cosmétiquement acceptable, on entend un milieu compatible avec toutes les matières kératiniques telles que la peau, les ongles, les cheveux, les cils et sourcils, les lèvres et toute autre zone cutanée du corps et du visage, mais aussi d'odeur, d'aspect et de toucher agréables.

La présente invention a donc pour objet l'utilisation en cosmétique en tant que gélifiant, d'au moins un polyorganosiloxane spécifique tel que décrit ci-dessous.

Un autre objet de la présente invention est une composition cosmétique comprenant, dans un milieu cosmétiquement acceptable, au moins un polyorganosiloxane spécifique tel que décrit ci-dessous..

D'autres caractéristiques, aspects et avantages de l'invention apparaîtront encore plus clairement à la lecture de la description et des divers exemples qui suivent.

Un objet de l'invention concerne l'utilisation en cosmétique en tant que gélifiant, d'au moins un organopolysiloxane, linéaire ou cyclique, qui comporte au moins deux motifs organosiloxy et au moins deux groupes latéraux et/ou terminaux capables d'établir chacun au moins une, de préférence au moins deux liaisons hydrogène avec un ou des groupes partenaires.

Les organopolysiloxanes convenant dans l'invention comprennent au moins deux motifs organosiloxy qui peuvent être notamment représentés par la formule suivante :

RₐR'_{b}SiO_{(4-a-b)/2} (I)

dans laquelle :
R représente un groupe alkyle linéaire, ramifié ou cyclique, un groupe aryle, un groupe polyéther ou un groupe fluoré,
R' représente un groupe capable d'établir au moins une liaison hydrogène, de préférence au moins deux liaisons hydrogène,
a vaut 1, 2 ou 3, et
b vaut 0 ou 1, à condition que a+b soit égal à 2 ou 3.

Le nombre desdits motifs organosiloxy va de préférence de 2 à 50 000, et mieux encore de 2 à 30 000.

Les groupes alkyle peuvent être linéaires, ramifiés ou cycliques, et choisis notamment parmi les groupes méthyle, éthyle, propyle, isopropyle, n-butyle, sec-butyle, tert-butyle, pentyle, cyclopentyle, cyclohexyle et autres analogues. Le groupe méthyle est particulièrement préféré.

Parmi les groupes aryle, on préfère le groupe phényle.

Comme exemples de groupe polyéther, on peut citer les groupes polyoxyéthylène, polyoxypropylène et polyoxyéthylène/polyoxypropylène.

Les groupes fluorés peuvent être des groupes alkyle linéaire, ramifié ou cyclique, ou alcényle, qui portent un ou plusieurs atomes de fluor comme substituants.

Les groupes R' sont des groupes latéraux et/ou terminaux capables d'établir des liaisons hydrogène et ils sont choisis de préférence parmi :
(a) les groupes dérivés d'acides aminés non protégés ou partiellement protégés, et
(b) les groupes acides carboxyliques, amines ou phénols de formule :

-X-(Y)ₙ-Z

dans laquelle :
X représente une chaîne d'espacement linéaire, ramifiée ou cyclique, de type alkylène ou alcénylène, comportant éventuellement un ou plusieurs hétéroatomes dans la chaîne,
Y représente un groupe hydrocarboné insaturé, divalent, monocyclique ou polycyclique, ces groupes polycycliques pouvant comporter jusqu'à 4 cycles condensés,
n représente un nombre entier allant de 1 à 4, et
Z représente un groupe -COOH, -OH ou un groupe amine primaire, secondaire ou tertiaire.

Comme cela est bien connu dans la technique, un groupe acide carboxylique peut former des liaisons hydrogène avec un autre groupe acide carboxylique ou un groupe amine, alors qu'un groupe amine peut former des liaisons hydrogène avec un groupe acide carboxylique ou un groupe -OH phénolique.

Ainsi, on peut utiliser dans la composition de l'invention, un unique organopolysiloxane ayant au moins deux groupes terminaux et/ou latéraux dont au moins l'un est un groupe -COOH et au moins un autre est un groupe -COOH ou amine (primaire, secondaire ou tertiaire), ou bien un unique organopolysiloxane ayant au moins deux groupes terminaux et/ou latéraux dont au moins l'un est un groupe amine (primaire, secondaire ou tertiaire) et au moins un autre est un groupe -OH phénolique ou -COOH.

On peut également utiliser des mélanges, de préférence équimolaires, de deux organopolysiloxanes comportant des groupes partenaires. Ainsi, on peut utiliser un mélange d'un organopolysiloxane ayant au moins deux groupes -COOH avec un organopolysiloxane ayant au moins deux groupes amines, ou bien un mélange d'un organopolysiloxane ayant au moins deux groupes amines et d'un organopolysiloxane ayant au moins deux groupes -OH phénoliques.

Les fonctions amine et/ou acide carboxylique des groupes dérivés d'acides aminés peuvent être non protégées ou partiellement protégées par des groupes particuliers tels que le groupe acétyle. Comme exemples de groupes dérivés d'acides aminés, on peut citer la cystéine, la N-acétylcystéine, le glycocolle, l'alanine, la sérine, la N-acétylcystéine étant particulièrement préférée.

La fonctionnalisation de l'organopolysiloxane par ces groupes dérivés d'acides aminés se fait par des techniques bien connues de l'homme de métier, telles que la silylation des liaisons insaturées par un dérivé thiol de l'acide aminé ou par réaction d'un organohydrogénosiloxane avec un dérivé d'acide aminé porteur d'une liaison insaturée.

La chaîne d'espacement X est un groupe alkylène ou alcénylène, linéaire, ramifié ou cyclique, qui peut comporter un ou plusieurs hétéroatomes tels que N, S ou O. A titre d'exemple de chaîne d'espacement, on peut citer -(CH₂)ₚ-S- ou -(CH₂)ₚ-O-, p variant de préférence de 1 à 5.

Parmi les groupes hydrocarbonés insaturés, divalents, monocycliques ou polycycliques, ou hétérocycliques insaturés, Y représente de préférence un noyau aromatique à 6 chaînons.

Comme groupe, hydrocarboné insaturé, divalent, monocyclique ou polycyclique, on peut citer les groupes phénylène ou naphtalène-diyle, le groupe phénylène étant particulièrement préféré.

Les organopolysiloxanes convenant dans la présente invention, sont les organopolysiloxanes linéaires ou cycliques, comportant au moins deux motifs organosiloxy et au moins deux groupes latéraux et/ou terminaux capables d'établir des liaisons hydrogène, tels que ceux décrits dans le document FR 2 708 272 cité ci-dessus.

L'organopolysiloxane préféré dans la présente invention répond à la formule suivante : 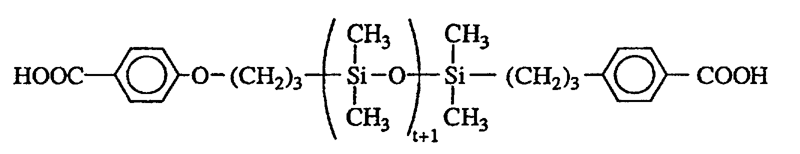 avec t allant de préférence de 1 à 1200, et notamment avec t=11.

Un autre objet de l'invention concerne une composition cosmétique comprenant, dans un milieu cosmétiquement acceptable, au moins un organopolysiloxane linéaire ou cyclique, qui comporte au moins deux motifs organosiloxy et au moins deux groupes latéraux et/ou terminaux capables d'établir chacun au moins une liaison hydrogène avec un ou des groupes partenaires, lesdits motifs organosiloxy étant représentés par la formule suivante :

RₐR'_{b}SiO_{(4-a-b)/2}

dans laquelle R, R', a et b sont tels que définis ci-dessus.

La préparation des organopolysiloxanes des compositions cosmétiques de l'invention est connue dans la technique. Des exemples de préparation sont décrits dans le brevet français numéro 2 708 272 de Rhône-Poulenc et dans l'article de S. Abed et al, Polym. Mater. Sci. Eng., 1997, N° 76, 45-46.

Un premier exemple de préparation consiste à utiliser un organopolysiloxane présentant des groupes latéraux insaturés, tels que des groupes vinyliques ou allyliques, et à le faire réagir avec un dérivé sulfanylique comme la N-acétylcystéine. On peut alors obtenir un organopolysiloxane comportant les motifs suivants : 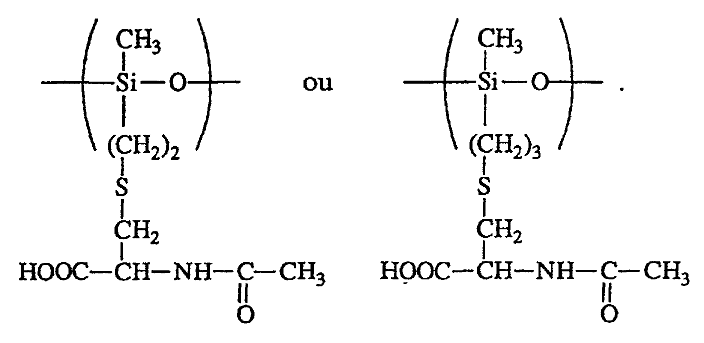 Un deuxième exemple de préparation consiste à utiliser un organopolysiloxane présentant des groupes silyle -SiH et à le faire réagir par hydrosilylation avec un dérivé d'acide aminé porteur d'une double liaison vinylique ou allylique, dont on a neutralisé par silylation les fonctions acide carboxylique et amine, comme par exemple : 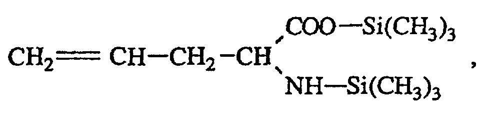 puis une fois la réaction d'hydrosilylation terminée, à déprotéger les fonctions acide carboxylique et amine. On obtient alors le même type de motifs que ceux représentés ci-dessus.

La synthèse d'organopolysiloxanes à groupes terminaux p-carboxyphényloxy est décrite dans l'article de S. Abed et al, Polym. Bull., 39, 1997, pages 317-324. Elle consiste à préparer tout d'abord un p-allyloxybenzoate de benzyle et à le faire réagir avec un organopolysiloxane à groupes terminaux -SiH par hydrosilylation. La dernière étape consiste à déprotéger les groupes terminaux pour obtenir finalement le produit suivant : 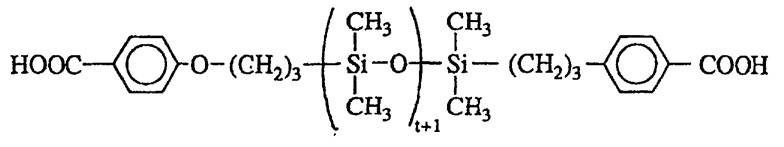 avec t allant de préférence de 1 à 1200.

Dans la composition cosmétique de l'invention, on utilise de préférence l'organopolysiloxane répondant à la formule ci-dessus avec t=11.

Les organopolysiloxanes de la présente invention sont notamment utilisés en une quantité se situant dans l'intervalle allant de 0,5 à 50 % en poids, de préférence de 1 à 30 % en poids par rapport au poids total de la composition cosmétique.

Le milieu cosmétiquement acceptable peut comprendre une phase grasse, éventuellement des solvants organiques et éventuellement de l'eau en une quantité telle qu'elle n'interfère pas avec les groupes du polyorganosiloxane pour la formation de liaisons hydrogène.

La phase grasse est notamment constituée de corps gras liquides à température ambiante (25 °C en général) et/ou de corps gras solides à température ambiante tels que les cires, les gommes, les corps gras pâteux et leurs mélanges.

Comme corps gras liquides à température ambiante, appelés souvent huiles, utilisables dans l'invention, on peut citer les huiles siliconées, hydrocarbonées, d'origine minérale, animale, végétale ou synthétique, seules ou en mélange dans la mesure où elles forment un mélange homogène et stable, et où elles sont compatibles avec l'utilisation envisagée.

Le milieu cosmétiquement acceptable contient de préférence des huiles siliconées volatiles et/ou non volatiles.

A titre d'huiles siliconées non volatiles, on peut citer les polydiméthylsiloxanes (PDMS), éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthyl-phénylsiloxanes, éventuellement substitués par des groupements aliphatiques et/ou aromatiques, ou éventuellement fluorés ; les poly-siloxanes modifiés par des acides gras, des alcools gras ou des polyoxyalkylènes, les silicones fluorées, les huiles siliconées perfluorées et leurs mélanges.

Parmi les huiles siliconées non volatiles préférées, on peut citer les polydiméthylsiloxanes, les polyméthylphénylsiloxanes, les silicones comportant des séquences ou des greffons polyoxyalkylènes, en particulier polyoxyéthylène ou copoly(oxyéthylène/oxypropylène) telles que les diméthiconecopolyols, les silicones portant à la fois des groupes hydrophobes hydrocarbonés (par exemple des groupes alkyle en C₂-C₃₀) et des séquences ou greffons polyoxyéthylénés ou copoly(oxyéthylénés/oxypropylénés) telles que les alkyldiméthiconecopolyols, les silicones portant des groupes fluorés ou perfluorés telles que les polydiméthylsiloxanes perfluoroalkylés et les polyméthylphénylsiloxanes perfluoroalkylés, et leurs mélanges.

On peut aussi utiliser de manière avantageuse une ou plusieurs huiles volatiles à température ambiante. Après évaporation de ces huiles, on obtient un dépôt filmogène souple. Ces huiles volatiles facilitent, en outre, l'application de la composition sur la peau, les lèvres et les phanères.

Par huile volatile, on entend une huile capable de s'évaporer à la température de la peau ou des lèvres, présentant une pression de vapeur non nulle à température ambiante et sous la pression atmosphérique, allant en particulier de 0,13 à 4,0 x 10⁴ Pa (10⁻³ à 300 mm Hg), et mieux encore supérieure à 40 Pa (0,3 mm Hg).

Ces huiles peuvent être des huiles de silicones comportant éventuellement des groupes alkyle ou alcoxy en bout de chaîne siliconée ou pendante.

Comme huile de silicone volatile utilisable dans l'invention, on peut citer les silicones linéaires ou cycliques, ayant une viscosité à température ambiante et sous pression atmosphérique inférieure à 8 mm²/s (8 cSt), et comportant en particulier de 2 à 7 atomes de silicium. On peut notamment citer l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et leurs mélanges.

De préférence, on utilise au moins une huile de silicone volatile choisie notamment parmi l'octaméthylcyclotétrasiloxane, le déca-méthylcyclopentasiloxane, l'hexadécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Le milieu cosmétiquement acceptable contenant une ou plusieurs huiles de silicone peut en outre contenir une ou plusieurs huiles de nature non siliconée comme, par exemple, des huiles hydrocarbonées.

Par huile hydrocarbonée, on entend une huile contenant majoritairement des atomes de carbone et d'hydrogène et notamment des chaînes alkyle ou alcényle comme les alcanes ou les alcènes, mais aussi une huile contenant en plus des atomes d'hydrogène et de carbone, des atomes d'oxygène sous forme de fonction éther, ester, alcool ou acide carboxylique.

On peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales; des esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle, le succinate de di(2-éthylhexyle), le malate de diisostéaryle, le triisostéarate de glycérine ou de diglycérine ; les alcools gras supérieurs à au moins 12 atomes de carbone tels que l'alcool stéarylique ou l'alcool oléique, l'alcool linoléique ou linolénique, l'alcool isostéarique ou l'octyldodécanol.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires pouvant être utilisées selon l'invention, on peut citer les cires d'origine animale telles que la cire d'abeilles, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple de paraffine, de vaseline, de lignite ou les cires microcristallines ou les ozokérites ; les cires synthétiques parmi lesquelles les cires de polyéthylène, de polytétrafluoroéthylène et les cires obtenues par synthèse de Fisher-Tropsch ou encore les cires de silicone, les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN.

Les gommes sont généralement des polydiméthylsiloxanes (PDMS) à haut poids moléculaire et les corps pâteux sont généralement des composés hydrocarbonés comme les lanolines et leurs dérivés, ou encore des PDMS.

La composition cosmétique selon l'invention peut également comprendre un ou plusieurs solvants organiques cosmétiquement acceptables (tolérance, toxicologie et toucher acceptables). Ces solvants organiques peuvent être choisis parmi les solvants organiques hydrophiles, les solvants organiques lipophiles, les solvants amphiphiles et leurs mélanges.

Parmi les solvants organiques hydrophiles, on peut citer, par exemple, des mono-alcools inférieurs, linéaires ou ramifiés, comportant de 1 à 8 atomes de carbone comme l'éthanol, le propanol, le butanol, l'isopropanol, l'isobutanol ; l'acétone ; des polyéthylèneglycols comportant de 6 à 80 motifs éthylèneoxy ; des polyols tels que le propylèneglycol, le butylèneglycol, le glycérol, le sorbitol ; les mono- ou di-alkylisosorbides dont les groupements alkyle comportent de 1 à 5 atomes de carbone comme le diméthyl-isosorbide; comme l'éther mono-méthylique ou mono-éthylique de diéthylèneglycol et les éthers de propylèneglycol comme l'éther méthylique de dipropylèneglycol.

Comme solvants organiques amphiphiles, on peut citer des polyols tels que des dérivés de polypropylèneglycol (PPG) comme les esters de polypropylèneglycol et d'acide gras, les éthers de PPG et d'alcool gras, par exemple, l'oléate de PPG-36 et l'éther oléylique de PPG-23.

Comme solvants organiques lipophiles, on peut citer, par exemple, les hydrocarbures tels que l'hexane, l'heptane et l'octane ; les esters gras tels que l'adipate de diisopropyle, l'adipate de dioctyle ; les benzoates d'alkyle ; le malate de dioctyle.

La composition cosmétique selon l'invention peut comprendre, en outre, au moins un ingrédient choisi parmi les actifs cosmétiques et/ou les actifs de soin selon le type d'application envisagée, ainsi que divers autres additifs classiques utilisés dans le domaine des cosmétiques, tels que, par exemple, des charges, des pigments, des colorants, des tensio-actifs, des filtres solaires, des anti-oxydants, des parfums, des conservateurs.

Les actifs cosmétiques et/ou de soin sont utilisés en une proportion habituelle pour l'homme de métier, et notamment en une proportion allant de 0,001 à 30 % en poids de la composition.

L'homme de métier veillera à choisir les éventuels additifs et leur quantité de manière à ce qu'ils ne nuisent pas aux propriétés des compositions cosmétiques, c'est-à-dire qu'ils ne doivent pas présenter de groupes fonctionnels capables de former des liaisons hydrogène avec les composants principaux de la composition cosmétique, qui ont été décrits ci-dessus.

Les charges peuvent être minérales ou organiques, lamellaires ou sphériques. On peut citer le talc, le mica, la silice, le kaolin, les poudres de Nylon® (Orgasol® d'Atochem), de poly-β-alanine et de polyéthylène, le Téflon®, la lauroyl-lysine, l'amidon, le nitrure de bore, les microsphères creuses telles que l'Expancel® (Nobel Industrie), le Polytrap® (Dow Corning) et les microbilles de résine de silicone (Tospearls® de Toshiba, par exemple), le carbonate de calcium précité, la carbonate et l'hydrocarbonate de magnésium, l'hydroxyapatite, les microsphères de silice creuses (Silica beads de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium.

Les pigments nacrés peuvent être choisis parmi les pigments nacrés blancs tels que le mica recouvert d'oxyde de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont, par exemple, le rouge Soudan, le DC Red 17, le DC Green 6, le β-carotène, le DC Yellow 11, le DC Violet 2. Ils peuvent représenter de 0,01 à 20 % du poids de la composition et mieux de 0,1 à 6 %.

Les tensio-actifs peuvent être des tensioactifs anioniques, cationiques ou non-ioniques.

Les filtres solaires sont choisis parmi les filtres solaires actifs dans l'UV-A et l'UV-B.

Dans un mode de réalisation particulier de l'invention, les compositions cosmétiques peuvent être préparées de manière usuelle par l'homme du métier et peuvent se présenter sous forme d'un produit coulé et, par exemple, sous la forme d'un stick ou bâton, ou sous la forme de coupelle utilisable par contact direct ou d'une éponge. En particulier, elles trouvent une application pour le maquillage et/ou le soin de la peau, des lèvres et des phanères, par exemple, en tant que fond de teint coulé, fard à joues ou à paupières coulé, rouge à lèvres, base ou baume de soin pour les lèvres, produit anti-cernes, déodorant et anti-transpirant, produit de maquillage du corps tel qu'un tatouage semi-permanent, produit solaire ou mascara pain. Elles peuvent aussi se présenter sous forme d'une pâte souple, de viscosité dynamique à 25°C de l'ordre de 1 à 40 Pa.s, telle que mesurée sur un appareil Haake RS 50, avec une viscosité extrapolée aux gradients de cisaillement inférieurs à 1 s⁻¹. Elles peuvent également se présenter sous forme d'une solution épaissie présentant une viscosité au moins double de celle de l'huile ou des huiles de base ne contenant pas de gélifiant.

Les compositions sont avantageusement anhydres et peuvent contenir jusqu'à 5% d'eau par rapport au poids total de la composition. Elles peuvent alors se présenter notamment sous forme de gel huileux, de liquide huileux ou huile, de pâte ou de stick. Ces différentes formes sont préparées selon les méthodes usuelles des domaines considérés.

Les exemples suivants illustrent la présente invention.

### Synthèse du composé A

Un polyorganosiloxane à groupes terminaux p-carboxyphényloxy tel que défini ci-dessus, est préparé selon le procédé décrit dans l'article de S. ABED, Polymer Bulletin, 39, 317-324 (1997). On obtient ainsi le composé A de formule suivante : 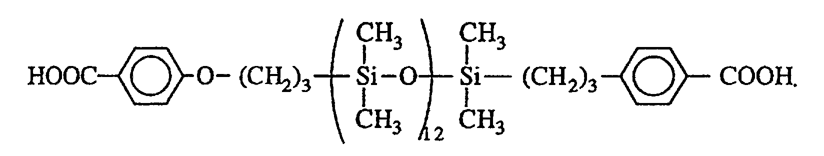 Ce composé A est utilisé dans les exemples suivants de préparation de rouge à lèvres et de brillant à lèvres.

### Exemple 1

On prépare un rouge à lèvres ayant la composition suivante :

| | |
|---|---|
| Cire de polyéthylène PERFORMALENE 500® ⁽¹⁾ | 15 g |
| Composé A | 5 g |
| Pigments | 9 g |
| Huile de polyisobutylène hydrogéné⁽²⁾ | 35,5 g |
| Huile de phényltriméthicone Dow 556 Fluid® ⁽³⁾ | 35,5 g |

| | |
|---|---|
| ⁽¹⁾ vendue par la société PETROLITE | |
| ⁽²⁾ de viscosité 34 mm²/s (34 cSt) à 25 °C, vendue sous le nom de "Parléam®" par la société NIPPON OIL-FATS. | |
| ⁽³⁾ vendue par la société DOW CORNING. | |

On mélange à 110 °C tous les constituants de la composition ci-dessus. Après homogénéisation et broyage des pigments, on coule le mélange dans un moule adéquat. On obtient ainsi un stick présentant de bonnes caractéristiques rhéologiques. Il permet de déposer sur les lèvres un film présentant une bonne tenue dans le temps.

### Exemple 2

On prépare un brillant à lèvres ayant la composition suivante :

| | |
|---|---|
| Composé A | 5 g |
| Pigment (DC Red N° 7 Calcium (laque)) | 5 g |
| Huile de phényltriméthicone Dow 556 Fluid® ^{(*)} | 90 g |

| | |
|---|---|
| ^{(*)} vendue par la société DOW CORNING. | |

Le composé A est tout d'abord dissous dans l'huile. On obtient un brillant à lèvres en dispersant les pigments dans cette phase grasse. Le brillant à lèvres ainsi obtenu peut être appliqué au pinceau sur les lèvres. Il confère une coloration brillante durable dans le temps.

### Exemple 3

On prépare un produit solaire anhydre ayant la composition suivante :

| | |
|---|---|
| Composé A | 5 g |
| TiO₂ nanométrique enrobé d'alumine et de stéarate d'aluminium (*) | 7 g |
| Huile de phényltriméthicone Dow 556 Fluid® ^{(**)} | qsp 100 g |

| | |
|---|---|
| ^{(*)} vendu sous la dénomination MT 100T par la société TAYCA | |
| ^{(**)} vendue par la société DOW CORNING. | |

Le composé A est tout d'abord dissous dans l'huile. On obtient un produit solaire en dispersant le pigment par broyage dans le milieu. Le produit solaire présente des propriétés de résistance à l'eau et au sébum.

## Revendications

1. Utilisation en cosmétique, en tant que gélifiant, d'au moins un organopolysiloxane, linéaire ou cyclique, qui comporte au moins deux motifs organosiloxy et au moins deux groupes latéraux et/ou terminaux capables d'établir chacun au moins une liaison hydrogène avec un ou des groupes partenaires, lesdits motifs organosiloxy étant représentés par la formule suivante
RₐR'_{b}SiO_{(4-a-b)/2}
dans laquelle :
R représente un groupe alkyle linéaire, ramifié ou cyclique, un groupe aryle, un groupe polyéther ou un groupe fluoré,
R' représente un groupe capable d'établir au moins une liaison hydrogène,
a vaut 1, 2 ou 3, et
b vaut 0 ou 1, à condition que a+b soit égal à 2 ou 3,
ledit groupe R' étant choisi parmi
(a) les groupes dérivés d'acides aminés non protégés ou partiellement protégés, et
(b) les groupes acides carboxyliques, amines ou phénols de formule :
-X-(Y)ₙ-Z
dans laquelle :
X représente une chaîne d'espacement linéaire, ramifiée ou cyclique, de type alkylène ou alcénylène, comportant éventuellement un ou plusieurs hétéroatomes dans la chaîne ;
Y représente un groupe hydrocarboné insaturé, divalent, monocyclique ou polycyclique, ces groupes polycycliques pouvant comporter jusqu'à 4 cycles condensés,
n représente un nombre entier allant de 1 à 4, et
Z représente un groupe -COOH, -OH, ou un groupe amine primaire, secondaire ou tertiaire.

2. Utilisation en cosmétique selon la revendication 1, **caractérisée en ce que** l'organopolysiloxane comporte de 2 à 50 000 motifs organosiloxy, et de préférence de 2 à 30 000 motifs organosiloxy.

3. Utilisation en cosmétique selon la revendication 1 ou 2, **caractérisée en ce que** les groupes latéraux et/ou terminaux de l'organopolysiloxane sont capables d'établir chacun au moins deux liaisons hydrogène avec un ou des groupes partenaires.

4. Utilisation en cosmétique selon l'une quelconque des revendications 1 à 3, **caractérisée en ce que** Y représente un noyau aromatique à 6 chaînons et Z représente un groupe -COOH.

5. Utilisation en cosmétique selon la revendication 4, **caractérisée en ce que** l'organopolysiloxane répond à la formule suivante : 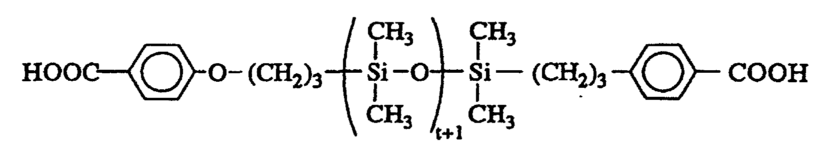 avec t allant de 1 à 1200.

6. Utilisation en cosmétique selon la revendication 5, **caractérisée en ce que** l'organopolysiloxane répond à la formule suivante :

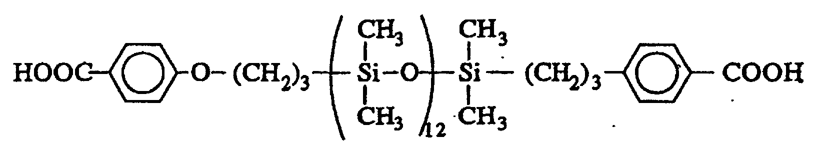

7. Composition cosmétique, **caractérisée en ce qu'**elle comprend, dans un milieu cosmétiquement acceptable, au moins un organopolysiloxane, linéaire ou cyclique, qui comporte au moins deux motifs organosiloxy et au moins deux groupes latéraux et/ou terminaux capables d'établir chacun au moins une liaison hydrogène avec un ou des groupes partenaires, lesdits motifs organosiloxy étant représentés par la formule suivante :
RₐR'_{b}SiO_{(4-a-b)/2}
dans laquelle :
R représente un groupe alkyle linéaire, ramifié ou cyclique, un groupe aryle, un groupe polyéther ou un groupe fluoré,
R' représente un groupe capable d'établir au moins une liaison hydrogène,
a vaut 1, 2 ou 3, et
b vaut 0 ou 1, à condition que a+b soit égal à 2 ou 3,
ledit groupe R' étant choisi parmi :
(a) les groupes dérivés d'acides aminés non protégés ou partiellement protégés, et
(b) les groupes acides carboxyliques, amines ou phénols de formule :
-X-(Y)ₙ-Z
dans laquelle :
X représente une chaîne d'espacement linéaire, ramifiée ou cyclique, de type alkylène ou alcénylène, comportant éventuellement un ou plusieurs hétéroatomes dans. la chaîne ;
Y représente un groupe hydrocarboné insaturé, divalent, monocyclique ou polycyclique, ces groupes polycycliques pouvant comporter jusqu'à 4 cycles condensés,
n représente un nombre entier allant de 1 à 4, et
Z représente un groupe-COOH, -OH, ou un groupe amine primaire, secondaire ou tertiaire.

8. Composition cosmétique selon la revendication 7, **caractérisée en ce que** l'organopolysiloxane comporte de 2 à 50 000 motifs organosiloxy, et de préférence de 2 à 30 000 motifs organosiloxy.

9. Composition cosmétique selon la revendication 7 ou 8, **caractérisée en ce que** les groupes latéraux et/ou terminaux de l'oragnopolysiloxane sont capables d'établir chacun au moins deux liaisons hydrogène avec un ou des groupes partenaires.

10. Composition cosmétique selon l'une quelconque des revendications 7 à 9, **caractérisée en ce que** Y représente un noyau aromatique à 6 chaînons et Z représente un groupe -COOH.

11. Composition cosmétique selon la revendication 10, **caractérisée en ce que** l'organopolysiloxane répond à la formule suivante : 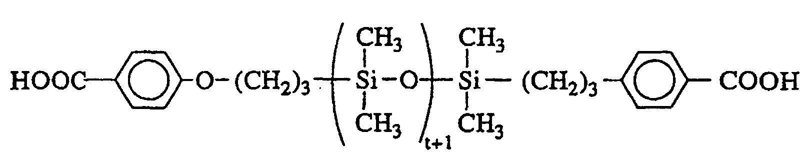 avec t allant de 1 à 1200.

12. Composition cosmétique selon la revendication 11, **caractérisée en ce que** l'organopolysiloxane répond à la formule suivante :

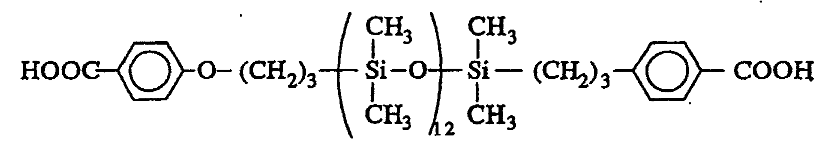

13. Composition cosmétique selon l'une quelconque des revendications 7 à 12, **caractérisée en ce que** la quantité de l'organopolysiloxane est comprise entre 0,5 et 50 % en poids, par rapport au poids total de la composition cosmétique.

14. Composition cosmétique selon la revendication 15, **caractérisée en ce que** la quantité de l'organopolysiloxane est comprise entre 1 et 30 % en poids, par rapport au poids total de la composition cosmétique.

15. Composition cosmétique selon l'une quelconque des revendications 7 à 14, **caractérisée en ce que** le milieu cosmétiquement acceptable comprend une phase grasse, éventuellement des solvants organiques et éventuellement de l'eau.

16. Composition cosmétique selon la revendication 15, **caractérisée en ce que** la phase grasse comprend des corps gras liquides à température ambiante et/ou des corps gras solides à température ambiante.

17. Composition cosmétique selon la revendication 16, **caractérisée en ce que** les corps gras liquides à température ambiante comprennent une huile de silicone et/ou une huile hydrocarbonée.

18. Composition cosmétique selon la revendication 17, **caractérisée en ce qu'**elle comprend au moins une huile de silicone.

19. Composition cosmétique selon la revendication 18, **caractérisée en ce que** l'huile de silicone est choisie parmi les polydiméthylsiloxanes (PDMS), éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthylphénylsiloxanes, éventuellement substitués par des groupements aliphatiques et/ou aromatiques, ou éventuellement fluorés ; les polysiloxanes modifiés par des acides gras, des alcools gras ou des polyoxyalkylènes, les silicones fluorées et les huiles siliconées perfluorées.

20. Composition cosmétique selon la revendication 17, **caractérisée en ce que** l'huile hydrocarbonée est choisie parmi l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales; les esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle, le succinate de di(2-éthylhexyle), le malate de diisostéaryle, le triisostéarate de glycérine ou de diglycérine ; les alcools gras supérieurs à au moins 12 atomes de carbone tels que l'alcool stéarylique ou l'alcool oléique, l'alcool linoléique ou linolénique, l'alcool isostéarique ou l'octyldodécanol.

21. Composition cosmétique selon la revendication 16, **caractérisée en ce que** les corps gras solides à température ambiante comprennent des cires, des gommes et/ou des corps gras pâteux.

22. Composition cosmétique selon la revendication 21, **caractérisée en ce que** les cires sont choisies parmi les cires d'origine animale, les cires végétales, les cires minérales, les cires synthétiques et les cires obtenues par synthèse de Fisher-Tropsch ou encore les cires de silicone, les huiles hydrogénées concrètes à 25°C.

23. Composition cosmétique selon la revendication 21, **caractérisée en ce que** les gommes sont choisies parmi les polydiméthylsiloxanes à haut poids moléculaire.

24. Composition cosmétique selon la revendication 21, **caractérisée en ce que** les corps gras pâteux sont choisis parmi les composés hydrocarbonés et les polydiméthylsiloxanes.

25. Composition cosmétique selon l'une quelconque des revendications 7 à 24, **caractérisée en ce qu'**elle comprend en outre des additifs choisis parmi les charges, les pigments, les colorants, les tensio-actifs, les filtres solaires, les anti-oxydants, les parfums et les conservateurs.

26. Composition cosmétique selon l'une quelconque des revendications 7 à 25, **caractérisée en ce qu'**elle est anhydre.

27. Composition cosmétique selon l'une quelconque des revendications 7 à 26, **caractérisée en ce qu'**elle se présente sous la forme d'un stick ou d'un bâton, sous la forme d'une pâte souple, de viscosité dynamique à 25 °C de l'ordre de 1 à 40 Pa.s, sous forme de coupelle, de gel huileux ou de liquide huileux.

28. Composition cosmétique selon l'une quelconque des revendications 7 à 27, **caractérisée en ce qu'**elle est utilisée pour le maquillage et/ou le soin de la peau, y compris celle des paupières, ainsi que des lèvres et des phanères.

29. Composition cosmétique selon l'une quelconque des revendications 7 à 28, **caractérisée en ce qu'**elle se présente sous la forme d'un rouge à lèvres, d'un mascara, d'un eye-liner, d'un fond de teint, d'une poudre, d'un fard à joues, d'ombre à paupières ou d'un maquillage de corps.

30. Composition cosmétique selon l'une quelconque des revendications 7 à 28, **caractérisée en ce qu'**elle se présente sous la forme d'un produit hydratant, d'un déodorant ou d'un anti-transpirant.

## Patentansprüche

1. Verwendung mindestens eines geradkettigen oder cyclischen Organopolysiloxans, das mindestens zwei Organosiloxyeinheiten und mindestens zwei Seitengruppen und/oder endständige Gruppen aufweist, die befähigt sind, jeweils mindestens eine Wasserstoff-Brückenbindung mit einem oder mehreren Bindungspartnern auszubilden, als Gelbildner in der Kosmetik, wobei die Organosiloxyeinheiten durch die folgende Formel dargestellt werden können:
RₐR'_{b}SiO_{(4-a-b)/2}
worin bedeuten:
R eine geradkettige, verzweigte oder cyclische Alkylgruppe, eine Arylgruppe, eine Polyethergruppe oder eine fluorierte Gruppe,
R' eine Gruppe, die befähigt ist, mindestens eine Wasserstoff-Brückenbindung auszubilden,
a 1, 2 oder 3, und
b 0 oder 1, mit der Maßgabe, dass a + b 2 oder 3 bedeutet, wobei die Gruppe R' ausgewählt ist unter:
(a) den Gruppen, die von nicht geschützten oder teilweise geschützten Aminosäuren abgeleitet sind, und
(b) den Carbonsäuregruppen, Aminogruppen oder Phenolgruppen der Formel:
-X-(Y)ₙ-Z
worin bedeuten:
X eine geradkettige, verzweigte oder cyclische Verbindungsgruppe vom Alkylentyp oder Alkenylentyp, die in der Kette gegebenenfalls ein oder mehrer Heteroatome enthält;
Y eine ungesättigte, zweiwertige, monocyclische oder polycyclische Kohlenwasserstoffgruppe, wobei die polycyclischen
Gruppen bis zu vier kondensierte Ringe aufweisen können, n eine ganze Zahl von 1 bis 4, und
Z die Gruppe -COOH, die Gruppe -OH oder eine primäre, sekundäre oder tertiäre Aminogruppe.

2. Verwendung in der Kosmetik nach Anspruch 1, **dadurch gekennzeichnet, dass** das Organopolysiloxan 2 bis 50 000 Organosiloxyeinheiten und vorzugsweise 2 bis 30 000 Organosiloxyeinheiten aufweist.

3. Verwendung in der Kosmetik nach Anspruch 1 oder 2, **dadurch gekennzeichnet, dass** die Seitengruppen und/oder endständigen Gruppen des Organopolysiloxans befähigt sind, jeweils mindestens zwei Wasserstoff-Brückenbindungen mit einem oder mehreren Bindungspartnern einzugehen.

4. Verwendung in der Kosmetik nach einem der Ansprüche 1 bis 3, **dadurch gekennzeichnet, dass** Y ein sechsgliedriger aromatischer Ring ist und Z die Gruppe -COOH bedeutet.

5. Verwendung in der Kosmetik nach Anspruch 4, **dadurch gekennzeichnet, dass** das Organopolysiloxan der folgenden Formel entspricht: 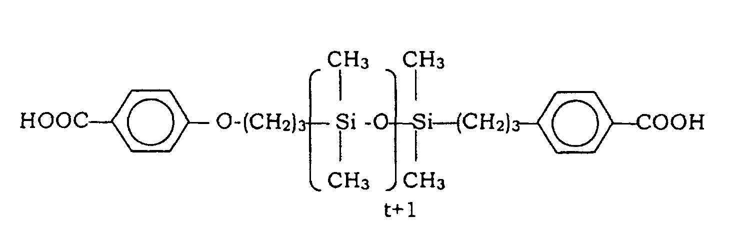 wobei t im Bereich von 1 bis 1200 liegt.

6. Verwendung in der Kosmetik nach Anspruch 5, **dadurch gekennzeichnet, dass** das Organopolysiloxan der folgenden Formel entspricht:

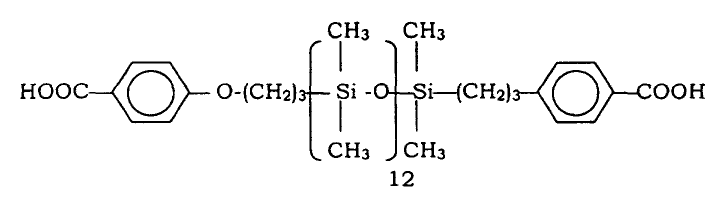

7. Kosmetische Zusammensetzung, **dadurch gekennzeichnet, dass** sie in einem kosmetisch akzeptablen Medium mindestens ein geradkettiges oder cyclisches Organopolysiloxan enthält, das mindestens zwei Organosiloxyeinheiten und mindestens zwei Seitengruppen und/oder endständige Gruppen aufweist, die befähigt sind, jeweils mindestens eine Wasserstoff-Brückenbindung mit einem oder mehreren Bindungspartnern einzugehen, wobei die Organosiloxyeinheiten durch die folgende Formel dargestellt werden können:
RₐR'_{b}SiO_{(4-a-b)/2}
worin bedeuten:
R eine geradkettige, verzweigte oder cyclische Alkylgruppe, eine Arylgruppe, eine Polyethergruppe oder eine fluorierte Gruppe, R' eine Gruppe, die befähigt ist, mindestens eine Wasserstoff-Brückenbindung auszubilden,
a 1, 2 oder 3, und
b 0 oder 1, mit der Maßgabe, dass a + b 2 oder 3 bedeutet, wobei die Gruppe R' ausgewählt ist unter:
(c) den Gruppen, die von nicht geschützten oder teilweise geschützten Aminosäuren abgeleitet sind, und
(d) den Carbonsäuregruppen, Aminogruppen oder Phenolgruppen der Formel:
-X-(Y)ₙ-Z
worin bedeuten:
X eine geradkettige, verzweigte oder cyclische Verbindungsgruppe vom Alkylentyp oder Alkenylentyp, die in der Kette gegebenenfalls ein oder mehrer Heteroatome enthält;
Y eine ungesättigte, zweiwertige, monocyclische oder polycyclische Kohlenwasserstoffgruppe, wobei die polycyclischen
Gruppen bis zu 4 kondensierte Ringe aufweisen können, n eine ganze Zahl von 1 bis 4, und
Z die Gruppe -COOH, die Gruppe -OH oder eine primäre, sekundäre oder tertiäre Aminogruppe.

8. Kosmetische Zusammensetzung nach Anspruch 7, **dadurch gekennzeichnet, dass** das Organopolysiloxan 2 bis 50 000 Organosiloxyeinheiten und vorzugsweise 2 bis 30 000 Organosiloxyeinheiten aufweist.

9. Kosmetische Zusammensetzung nach Anspruch 7 oder 8, **dadurch gekennzeichnet, dass** die Seitengruppen und/oder endständigen Gruppen des Organopolysiloxans befähigt sind, jeweils mindestens zwei Wasserstoff-Brückenbindungen mit einem oder mehreren Bindungspartnern auszubilden.

10. Kosmetische Zusammensetzung nach einem der Ansprüche 7 bis 9, **dadurch gekennzeichnet, dass** Y einen sechsgliedrigen aromatischen Ring und Z die Gruppe -COOH bedeutet.

11. Kosmetische Zusammensetzung nach Anspruch 10, **dadurch gekennzeichnet, dass** das Organopolysiloxan der folgenden Formel entspricht: 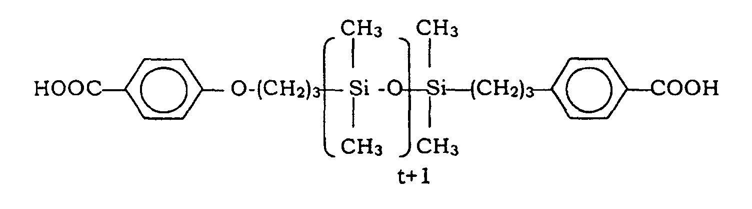 wobei t im Bereich von 1 bis 1200 liegt.

12. Kosmetische Zusammensetzung nach Anspruch 11, **dadurch gekennzeichnet, dass** das Organopolysiloxan der folgenden Formel entspricht:

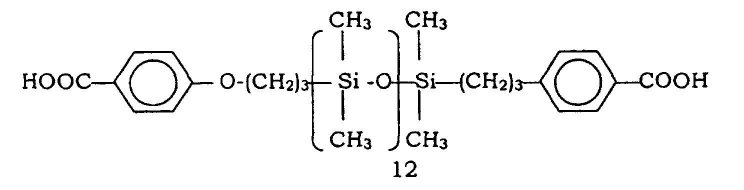

13. Kosmetische Zusammensetzung nach einem der Ansprüche 7 bis 12, **dadurch gekennzeichnet, dass** die Menge des Organopolysiloxans im Bereich von 0,5 bis 50 Gew.-%, bezogen auf das Gesamtgewicht der kosmetischen Zusammensetzung, liegt.

14. Kosmetische Zusammensetzung nach Anspruch 15, **dadurch gekennzeichnet, dass** der Mengenanteil des Organopolysiloxans im Bereich von 1 bis 30 Gew.-%, bezogen auf das Gesamtgewicht der kosmetischen Zusammensetzung, liegt.

15. Kosmetische Zusammensetzung nach einem der Ansprüche 7 bis 14, **dadurch gekennzeichnet, dass** das kosmetisch akzeptable Medium eine Fettphase, gegebenenfalls organische Lösungsmittel und gegebenenfalls Wasser enthält.

16. Kosmetische Zusammensetzung nach Anspruch 15, **dadurch gekennzeichnet, dass** die Fettphase bei Umgebungstemperatur flüssige Fettsubstanzen und/oder bei Umgebungstemperatur feste Fettsubstanzen enthält.

17. Kosmetische Zusammensetzung nach Anspruch 16, **dadurch gekennzeichnet, dass** die bei Umgebungstemperatur flüssigen Fettsubstanzen ein Siliconöl und/oder ein Kohlenwasserstofföl umfassen.

18. Kosmetische Zusammensetzung nach Anspruch 17, **dadurch gekennzeichnet, dass** sie mindestens ein Siliconöl enthält.

19. Kosmetische Zusammensetzung nach Anspruch 18, **dadurch gekennzeichnet, dass** das Siliconöl unter den Polydimethylsiloxanen (PDMS), die gegebenenfalls phenyliert sind, wie beispielsweise Phenyltrimethiconen, Phenyltrimethylsiloxydiphenylsiloxanen, Diphenylmethyldimethyltrisiloxanen, Diphenyldimethiconen, Phenyldimethiconen, Polymethylphenylsiloxanen, die gegebenenfalls mit aliphatischen und/oder aromatischen Gruppen substituiert oder gegebenenfalls fluoriert sind; den Polysiloxanen, die mit Fettsäuren, Fettalkoholen oder Polyalkylenoxiden modifiziert sind, den fluorierten Siliconen und den perfluorierten Siliconölen ausgewählt ist.

20. Kosmetische Zusammensetzung nach Anspruch 17, **dadurch gekennzeichnet, dass** das Kohlenwasserstofföl ausgewählt ist unter Paraffinöl, Vaselineöl, Nerzöl, Schildkrötenöl, Sojaöl, Perhydrosqualen, Süßmandelöl, Calophyllumöl, Palmöl, Traubenkernöl, Sesamöl, Maisöl, Parleam, Araraöl, Rapsöl, Sonnenblumenöl, Baumwollöl, Aprikosenöl, Ricinusöl, Avocadoöl, Jojobaöl, Olivenöl oder Getreidekeimöl; Estern von Lanolinsäure, Ölsäure, Laurinsäure oder Stearinsäure; Fettsäureestern, wie Isopropylmyristat, Isopropylpalmitat, Butylstearat, Hexyllaurat, Diisopropyladipat, Isononylisononanoat, 2-Ethylhexylpalmitat, 2-Hexyldecyllaurat, 2-Octyldecylpalmitat, 2-Octyldodecylmyristat, 2-Octyldodecyllactat, Di-(2-ethylhexyl)succinat, Diisostearylmalat, Glyceryltriisostearat oder Diglyceryltriisostearat; und höheren Fettalkoholen mit mindestens 12 Kohlenstoffatomen, wie Stearylalkohol, Oleylalkohol, Linoleylalkohol, Linolenylalkohol, Isostearylalkohol oder Octyldodecanol.

21. Kosmetische Zusammensetzung nach Anspruch 16, **dadurch gekennzeichnet, dass** die bei Umgebungstemperatur festen Fettsubstanzen Wachse, Gummis und/oder pastöse Fettsubstanzen umfassen.

22. Kosmetische Zusammensetzung nach Anspruch 21, **dadurch gekennzeichnet, dass** die Wachse unter den Wachsen tierischer Herkunft, pflanzlichen Wachsen, mineralischen Wachsen, synthetischen Wachsen und durch Fischer-Tropsch-Synthese hergestellten Wachsen oder Siliconwachsen und bei 25° C festen, hydrierten Ölen ausgewählt sind.

23. Kosmetische Zusammensetzung nach Anspruch 21, **dadurch gekennzeichnet, dass** die Gummis unter den Polydimethylsiloxanen mit hohem Molekulargewicht ausgewählt sind.

24. Kosmetische Zusammensetzung nach Anspruch 21, **dadurch gekennzeichnet, dass** die pastösen Fettsubstanzen unter den Kohlenwasserstoffverbindungen und den Polydimethylsiloxanen ausgewählt sind.

25. Kosmetische Zusammensetzung nach einem der Ansprüche 7 bis 24, **dadurch gekennzeichnet, dass** sie ferner Zusatzstoffe enthält, die unter den Füllstoffen, Pigmenten, Farbstoffen, grenzflächenaktiven Stoffen, Sonnenschutzfiltern, Antioxidantien, Parfüms und Konservierungsmitteln ausgewählt sind.

26. Kosmetische Zusammensetzung nach einem der Ansprüche 7 bis 25, **dadurch gekennzeichnet, dass** sie wasserfrei ist.

27. Kosmetische Zusammensetzung nach einem der Ansprüche 7 bis 26, **dadurch gekennzeichnet, dass** sie als Stift oder Stick, in Form einer weichen Paste mit einer dynamischen Viskosität in der Größenordnung von 1 bis 40 Pa·s bei 25° C, in Tiegelform oder als öliges Gel oder ölige Flüssigkeit vorliegt.

28. Kosmetische Zusammensetzung nach einem der Ansprüche 7 bis 27, **dadurch gekennzeichnet, dass** sie zum Schminken und/oder zur Pflege der Haut einschließlich der Lider sowie der Lippen und der Hautanhangsgebilde verwendet wird.

29. Kosmetische Zusammensetzung nach einem der Ansprüche 7 bis 28, **dadurch gekennzeichnet, dass** sie als Lippenstift, Mascara, Eyeliner, Make-up, Puder, Wangenrouge, Lidschatten oder Zusammensetzung zum Schminken des Körpers vorliegt.

30. Kosmetische Zusammensetzung nach einem der Ansprüche 7 bis 28, **dadurch gekennzeichnet, dass** sie in Form eines hydratisierenden Produktes, als Deodorant oder als Antitranspirant vorliegt.

## Claims

1. Cosmetic use, as a gelling agent, of at least one linear or cyclic polyorganosiloxane which comprises at least two organosiloxy units and at least two side and/or end groups each capable of forming at least one hydrogen bond with one or more partner groups, the said organosiloxy units being represented by the following formula:
Rₐ R'_{b}SiO_{(4-a-b)/2}
in which:
R represents a linear, branched or cyclic alkyl group, an aryl group, a polyether group or a fluoro group,
R' represents a group capable of forming at least one hydrogen bond,
a is 1, 2 or 3, and
b is 0 or 1, with the proviso that a+b is equal to 2 or 3, the said group R' being chosen from:
(a) groups derived from unprotected or partially protected amino acids, and
(b) carboxylic acid, amine or phenol groups of formula:
-X-(Y)ₙ-Z
in which:
X represents a linear, branched or cyclic spacer chain, of alkylene or alkenylene type, optionally comprising one or more hetero atoms in the chain,
Y represents a monocyclic or polycyclic divalent unsaturated hydrocarbon-based group, these polycyclic groups possibly comprising up to 4 fused rings,
n represents an integer ranging from 1 to 4, and
Z represents a -COOH or -OH group or a primary, secondary or tertiary amine group.

2. Cosmetic use according to Claim 1, **characterized in that** the polyorganosiloxane contains from 2 to 50,000 organosiloxy units and preferably from 2 to 30,000 organosiloxy units.

3. Cosmetic use according to Claim 1 or 2, **characterized in that** the side and/or end groups of the polyorganosiloxane are each capable of forming at least two hydrogen bonds with one or more partner groups.

4. Use in cosmetics according to any one of Claims 1 to 3, **characterized in that** Y represents a 6-membered aromatic nucleus and Z represents a -COOH group.

5. Use in cosmetics according to Claim 4, **characterized in that** the polyorganosiloxane corresponds to the following formula: 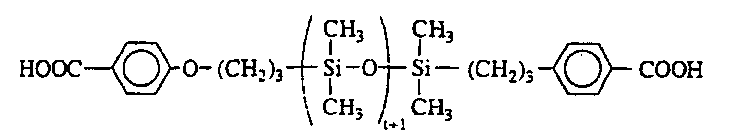 with t ranging from 1 to 1200.

6. Use in cosmetics according to Claim 5, **characterized in that** the polyorganosiloxane corresponds to the following formula:

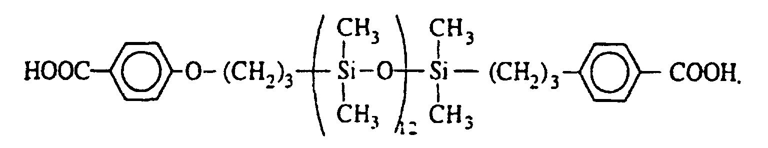

7. Cosmetic composition, **characterized in that** it comprises, in a cosmetically acceptable medium, at least one linear or cyclic polyorganosiloxane, which comprises at least two organosiloxy units and at least two side and/or end groups which are each capable of forming at least one hydrogen bond with one or more partner groups, the said organosiloxy units being represented by the following formula:
Rₐ R'_{b}SiO_{(4-a-b)/2}
in which:
R represents a linear, branched or cyclic alkyl group, an aryl group, a polyether group or a fluoro group,
R' represents a group capable of forming at least one hydrogen bond,
a is 1, 2 or 3, and
b is 0 or 1, with the proviso that a+b is equal to 2 or 3,
the said group R' being chosen from:
(a) groups derived from unprotected or partially protected amino acids, and
(b) carboxylic acid, amine or phenol groups of formula:
-X-(Y)ₙ-Z
in which:
X represents a linear, branched or cyclic spacer chain, of alkylene or alkenylene type, optionally comprising one or more hetero atoms in the chain,
Y represents a monocyclic or polycyclic divalent unsaturated hydrocarbon-based group, these polycyclic groups possibly comprising up to 4 fused rings,
n represents an integer ranging from 1 to 4, and
Z represents a -COOH or -OH group or a primary, secondary or tertiary amine group.

8. Cosmetic composition according to Claim 7, **characterized in that** the polyorganosiloxane comprises from 2 to 50,000 organosiloxy units and preferably from 2 to 30,000 organosiloxy units.

9. Cosmetic composition according to Claim 7 or 8, **characterized in that** the side and/or end groups of the polyorganosiloxane are each capable of forming at least two hydrogen bonds with one or more partner groups.

10. Cosmetic composition according to any one of Claims 7 to 9, **characterized in that** Y represents a 6-membered aromatic nucleus and Z represents a -COOH group.

11. Cosmetic composition according to Claim 10, **characterized in that** the polyorganosiloxane corresponds to the following formula: 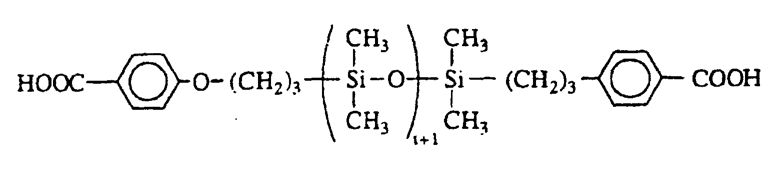 with t ranging from 1 to 1200.

12. Cosmetic composition according to Claim 11, **characterized in that** the polyorganosiloxane corresponds to the following formula:

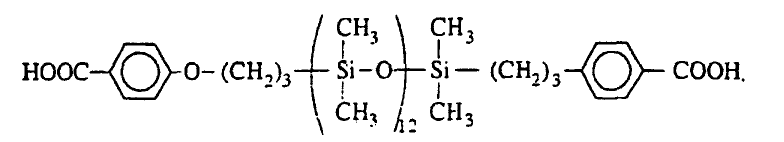

13. Cosmetic composition according to any one of Claims 7 to 12, **characterized in that** the amount of the polyorganosiloxane is between 0.5% and 50% by weight relative to the total weight of the cosmetic composition.

14. Cosmetic composition according to Claim 15 [sic], **characterized in that** the amount of the polyorganosiloxane is between 1% and 30% by weight relative to the total weight of the cosmetic composition.

15. Cosmetic composition according to any one of Claims 7 to 14, **characterized in that** the cosmetically acceptable medium comprises a fatty phase, optionally organic solvents and optionally water.

16. Cosmetic composition according to Claim 15, **characterized in that** the fatty phase comprises fatty substances that are liquid at room temperature and/or fatty substances that are solid at room temperature.

17. Cosmetic composition according to Claim 16, **characterized in that** the fatty substances that are liquid at room temperature comprise a silicone oil and/or a hydrocarbon-based oil.

18. Cosmetic composition according to Claim 17, **characterized in that** it comprises at least one silicone oil.

19. Cosmetic composition according to Claim 18, **characterized in that** the silicone oil is chosen from polydimethylsiloxanes (PDMSs), that are optionally phenylated, such as phenyltrimethicones, phenyltrimethylsiloxydiphenylsiloxanes, diphenylmethyldimethyltrisiloxanes, diphenyldimethicones, phenyldimethicones, polymethylphenylsiloxanes, optionally substituted with aliphatic and/or aromatic groups, or optionally fluorinated; polysiloxanes modified with fatty acids, fatty alcohols or polyoxyalkylenes, fluorosilicones and perfluorosilicone oils.

20. Cosmetic composition according to Claim 17, **characterized in that** the hydrocarbon-based oil is chosen from liquid paraffin, liquid petroleum jelly, mink oil, turtle oil, soybean oil, perhydrosqualene, sweet almond oil, beauty-leaf oil, palm oil, grape pip oil, sesame oil, corn oil, parleam oil, arara oil, rapeseed oil, sunflower oil, cottonseed oil, apricot oil, castor oil, avocado oil, jojoba oil, olive oil or cereal germ oil; esters of linoleic acid, of oleic acid, of lauric acid or of stearic acid; fatty esters, such as isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, bis(2-ethylhexyl) succinate, diisostearyl malate, glyceryl triisostearate or diglyceryl triisostearate; higher fatty alcohols containing at least 12 carbon atoms, such as stearyl alcohol, oleyl alcohol, linoleyl alcohol, linolenyl alcohol, isostearyl alcohol or octyldodecanol.

21. Cosmetic composition according to Claim 16, **characterized in that** the fatty substances that are solid at room temperature comprise waxes, gums and/or pasty fatty substances.

22. Cosmetic composition according to Claim 21, **characterized in that** the waxes are chosen from waxes of animal origin, plant waxes, mineral waxes, synthetic waxes and the waxes obtained by Fisher-Tropsch synthesis, or alternatively silicone waxes and hydrogenated oils that are solid at 25°C.

23. Cosmetic composition according to Claim 21, **characterized in that** the gums are chosen from high molecular weight polydimethylsiloxanes.

24. Cosmetic composition according to Claim 21, **characterized in that** the pasty fatty substances are chosen from hydrocarbon-based compounds and polydimethylsiloxanes.

25. Cosmetic composition according to any one of Claims 7 to 24, **characterized in that** it also comprises additives chosen from fillers, pigments, colourants, surfactants, sunscreens, antioxidants, fragrances and preserving agents.

26. Cosmetic composition according to any one of Claims 7 to 25, **characterized in that** it is anhydrous.

27. Cosmetic composition according to any one of Claims 7 to 26, **characterized in that** it is in the form of a stick or tube, in the form of a soft paste, with a dynamic viscosity at 25°C of about from 1 to 40 Pa.s, or in the form of a dish, an oily gel or an oily liquid.

28. Cosmetic composition according to any one of Claims 7 to 27, **characterized in that** it is used for making up and/or caring for the skin, including the skin of the eyelids, and also the lips and superficial body growths.

29. Cosmetic composition according to any one of Claims 7 to 28, **characterized in that** it is in the form of a lipstick, a mascara, an eyeliner, a foundation, a powder, a blusher, an eyeshadow or a body make-up.

30. Cosmetic composition according to any one of Claims 7 to 28, **characterized in that** it is in the form of a moisturizing product, a deodorant or an antiperspirant.
